# EUROPEAN PATENT SPECIFICATION

(11) **EP 2 004 608 B1**
(45) Date of publication and mention of the grant of the patent: **14.09.2011**
(21) Application number: 06808904.4
(22) Date of filing: 28.09.2006
(51) Int. Cl.: C07D 215/18

(54) **AN IMPROVED PROCESS FOR THE MANUFACTURE OF MONTELUKAST SODIUM**
VERBESSERTES VERFAHREN ZUR HERSTELLUNG VON MONTELUKAST-NATRIUM
PROCÉDÉ AMÉLIORÉ POUR LA FABRICATION DE MONTÉLUKAST SODIQUE

(30) Priority: 12.04.2006 IN MU05742006
(43) Date of publication of application: 24.12.2008
(73) Proprietor: Glade Organics Private Limited, Mumbai 400 054 (IN)
(72) Inventor: CHAWLA, Harmander, Pal, Singh, Ahmedabad (IN); CHOWDHARY, Anil, Shankar, Ahmedabad 380 015 (IN); PATEL, Ajay, Mangubhai, Gujarat 396 055 (IN); PATEL, Manish, Popatlal, Ahmedabad 380 043 (IN)
(74) Representative: Fiussello, Francesco
(86) International application number: PCT/IB2006/002690
(87) International publication number: WO 2007/116240

(56) References cited:
- EP-B1- 0 737 186
- WO-A1-2004/108679
- WO-A1-2006/058545
- WO-A2-2006/008751
- WO-A2-2006/064269
- US-A1- 2005 107 612

## Description

### BACKGROUND OF THE INVENTION

The present invention relates to an improved process for the manufacture of 1-[[[(1R)-1-[3-[(1E)-2-(7-chloro-2-quinolinyl)ethenyl] phenyl]-3-[2-(1-hydroxy-1-methylethyl) phenyl] propyl] thio] methyl] cyclopropane acetic acid, sodium salt I, which is known as Montelukast sodium.

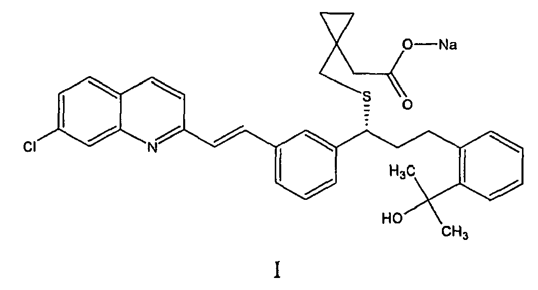

The compound of the formula I is a selective and orally active leukotriene receptor antagonist that inhibits the cysteinyl leukotriene CysLT₁ receptor. These compounds are effective in the treatment of asthmatic disorders, etc. Several processes for the manufacture of the same are reported.

### PRIOR ART

The European Patent No. 480717 discloses a class of novel anti-asthmatic compounds including montelukast sodium of structural formula I, having activity as leukotriene antagonists and to methods for their preparation. This patent provided a process for the preparation of the title compound I, which comprises of converting an alcohol of the formula II to a mesylate of the formula III. The mesylate is then condensed, in presence of cesium carbonate, with methyl (1-acetylthiomethyl) cyclopropaneacetate of the formula IV, after treatment of the latter with hydrazine, to obtain a compound of the formula V

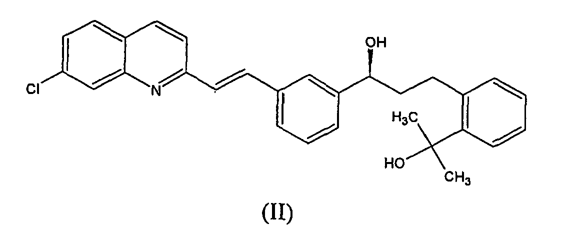

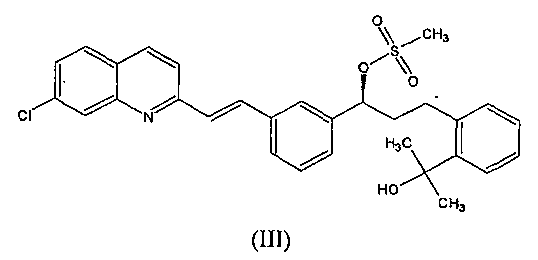

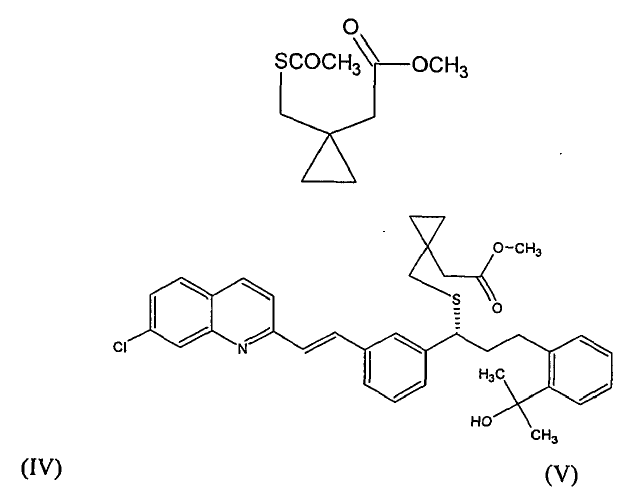

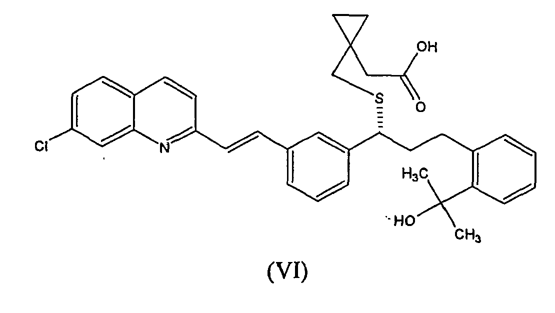

Finally the acid of the formula VI was prepared by hydrolysis of the methyl ester V in presence of pyridinium p-toluenesulphonate. The acid VI was, then, taken up in ethanol, treated with an equivalent amount of NaOH and the resultant oil was freeze dried to afford the compound of the formula I. This process afforded the title compound in low yields and purities and required purification by chromatography at intermediate stages.

European Patent No. 500360 relates to quinoline-containing ketoacids having activity as leukotriene antagonists and to methods for their preparation. This patent again provides processes as exemplified in EP 480717 and hence suffers from the same drawbacks.

The European Patent No. 737186 relates to a process for the preparation of a compound of the formula I which comprises of reacting the dilithium dianion of 1-(mercaptomethyl) cyclopropaneacetic acid (VII) with methanesulphonyloxy compound of the formula III to afford after suitable workup the acid VI which was in situ converted to its dicyclohexyl amine (DCHA) salt having the formula VIII.

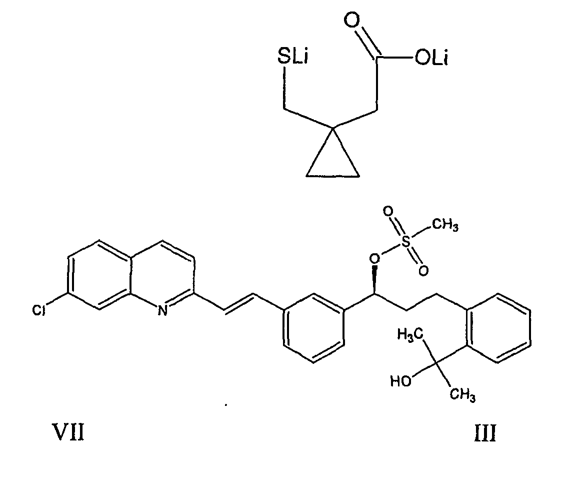

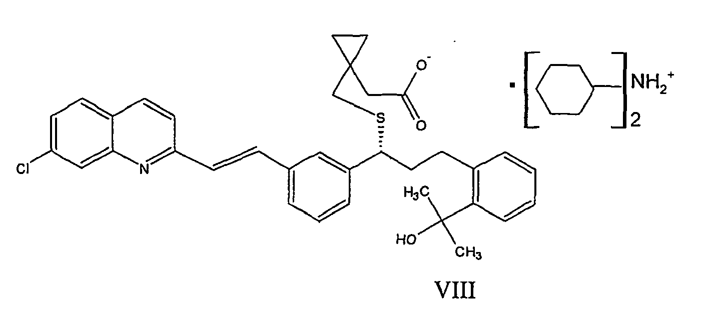

The dicyclohexylamine salt was purified by leaching with solvents and dried. The dried salt VIII was taken up in toluene and treated with acetic acid to generate free acid VI, the toluene solution of which was subsequently treated with an equivalent quantity of sodium hydroxide and the sodium salt so formed (I) was crystallized from a solvent mixture comprising of toluene-acetonitrile. This process suffers from multiplicity of steps involving formation of VI, conversion of the latter to its dicyclohexylamine salt, purification of the dicyclohexylamine salt, regeneration of acid VI before it is converted to montelukast sodium which is crystallized, making it very tedious and industrially unattractive.

The provisional patent application WO 03/066598 discloses an anhydrous amorphous form of montelukast sodium of the formula I which comprises of preparing the montelukast free acid from montelukast dicyclohexylamine salt by acidification, dissolving the free acid of montelukast in a C₁-C₂ halogenated solvent or in C₇-C₈ aromatic hydrocarbon solvent and converting the dissolved acid to the corresponding alkali salt using an alkaline metal hydroxide/an alkaline metal alkoxide/alcoholic alkaline metal hydroxide/ alcoholic alkaline metal alkoxide in presence of C₁-C₄ straight or branched chain alcohol and isolating amorphous form of montelukast alkali salt by adding a C₅-C₇ acyclic or C₅-C₈ cyclic hydrocarbon. This process affords the compound of the formula in yields less than 70% of theory, which renders the process unattractive.

The provisional patent application WO 04/108679 relates to an improved method for the preparation of montelukast acid sodium salt in an amorphous form which comprises of generating the dilithium dianion of 1-(thiomethyl)cyclopropaneacetic acid (VII) and coupling said dianion with wet mesylate of the formula III to get montelukast acid VI in crude form followed by conversion of the latter to its DCHA salt, purifying the DCHA salt and converting the DCHA salt to montelukast acid in the pure form and finally reacting the pure montelukast acid with a sodium base followed by evaporation of the solvent and triturating the residue with nonpolar water immiscible solvent to obtain the title compound, I. This long drawn procedure affects the overall yield of the final product.

The US application US2005/0187245 discloses a stable non-hygroscopic amorphous form of the compound of the formula I, which comprises of dissolving the montelukast sodium in a solvent/ a combination of solvents followed by spray drying the resultant solution. As a comparative example the patent also reports that a product prepared according to the process disclosed in EP 480717 which comprises of freeze drying an aqueous solution of montelukast sodium, provides an amorphous form as confirmed by the X-ray Diffraction data of the product. The patent does not disclose the yields obtained by following the procedure and is thus not clear.

Other process to prepare Montelukast salt are disclosed in WO 2006/058545, WO 2006/064269 and US 2005/0107612.

### SUMMARY

It is an objective of the present invention to provide a process for the manufacture of the compound of formula I in good yields by reducing the number of steps while still achieving good purities.

The process of the present invention utilizes 3 novel concepts for the manufacture of the compound of the formula I-
1. An important concept of the present invention is to utilize the ease of isolation of metal carboxylate salts wherein an ester compound of the formula IX is hydrolytically converted to a monometal salt of the formula X that can be isolated by filtration and thereafter can be dried to desired limits. These mono metallides can thereafter be converted to the dimetallides by use of a metal hydride, metal alkyl derivatives etc. This step serves the function of converting the mercapto end of 1-(mercaptomethyl)-cyclopropane acetate metal salt, X to its dimetal salt, XI. Thus the process of the present invention utilizes lesser quantities of metal alkyl derivatives.
2. A very important concept of the present invention is to convert an alcohol derivative and of the formula II to alkyl sulfonate compounds of the formula III, which are, reacted in-situ with the compound of the formula XI. It is well known to those conversant in the art that compounds of the formula III are relatively unstable and their isolation by operations such as filtration etc becomes an industrially critical operation which need special handling systems and hence the process of the present invention provides an efficient and hitherto unreported method of utilizing in-situ the thus obtained alkyl sulfonate which thereby affords much improved yields and also makes the process industrially easy to carry out. Thus the process for the manufacture of the compound of the formula I is rendered simple, easy and convenient to carry out on a large scale.
3. Another important concept of the present invention is to provide the synthetic utility of bases particularly the chirally pure bases such as α-methyl benzylamine, brucine, strychnine, quinine, cinchonidine, ephedrine, amphetamine, phenylpropanol amine etc for isolation and purification of the respective salts of montelukast. These chiral bases afford the title compound in better efficiencies and purities which thereby affords a process for the manufacture of the compound of the formula I that is highly economical and commercially advantageous.

### DETAILED DESCRIPTION

In an attempt to devise a more efficient process for montelukast sodium (I), it was conceived that the ester of the formula IX that has been reported in EP480717 could be readily converted to metal salts (X). These metal salts of the formula X apart from protecting the carboxylic acid can also be isolated as stable crystalline salts, which can be characterized. These mono metallides can be reacted with anhydrous metallide forming reagents affording the dimetallide derivatives, which can have potential uses for onward coupling with suitable substrates. 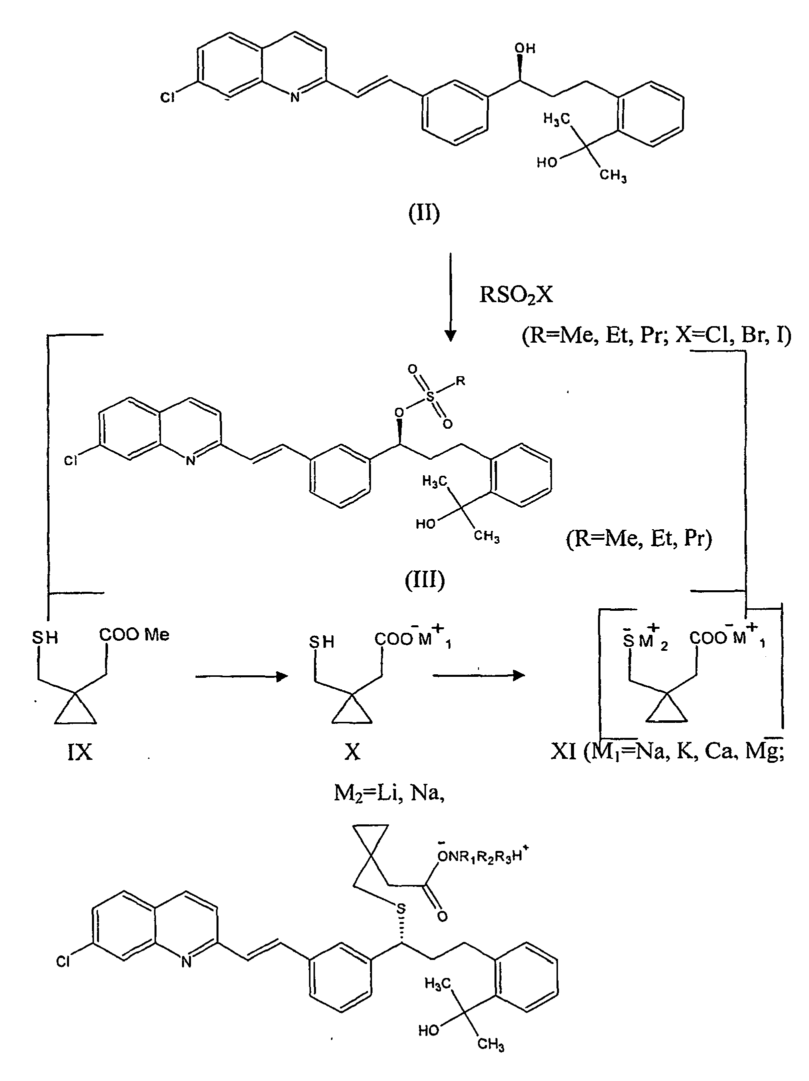 XII (R₁,R₂,R₃=H, achiral or chiral alkyl, cycloalkyl, heteroalkyl or heterocycloalkyl)

According to the invention there is provided a process for the manufacture of the compound of the formula I consisting of converting methyl 1-(mercaptomethyl)-cyclopropane acetate of the formula IX to metal salts of the formula X in a suitable solvent at 0 to 50°C wherein M₁ can be alkali metal or an alkaline earth metal such as Na⁺, K⁺, Ca⁺², Mg⁺² etc. The compounds of the formula X are dried and characterized. These are then converted to the dimetal salt of the formula XI in a suitable solvent at 0 to -50°C wherein M₁ is as described above and M₂ is an alkali metal such as Li⁺, Na⁺, K⁺ etc. The process is so carried out that simultaneously as XI is being synthesized, a compound of the formula II is converted to compounds of the formula III. This alkyl sulfonation affords compounds of the formula III which is monitored by HPLC (herein described in the examples) which without isolation are condensed "in-situ" with the simultaneously prepared compounds of the formula XI in a suitable solvent at 0 to - 50°C. This reaction is again monitored by HPLC. After the specified limits are achieved the reaction mass is quenched and extracted with a suitable solvent. The organic layer is thereafter treated with a suitable base, preferably a chirally pure base. This affords the crystallization of montelukast salt (XII) with a base preferably a chirally pure base, which is isolated by filtration. The compound of the formula XII is then purified by crystallization from a suitable solvent. It was important to establish that during the in-situ condensation of alkyl sulfonate III with the dimetalide XI, complete inversion occurs at the carbon carrying the alkyl sulfonate group, to give the desired enantiomer XII and that the proportion of the undesired enantiomer XIII does not increase as compared to the standard procedure reported in EP 737186. This was done by preparing XII where the chiral base used is (R)-(+)-α-methylbenzyl amine and also preparing (R)-(+)-α-methylbenzyl amine salt of montelukast obtained according to the procedure described in EP 737186 wherein instead of adding DCHA as prescribed in the process α-methyl benzylamine was added. The specific optical rotations of the two salts were comparable.

To obtain montelukast sodium (I), the purified salt XII is dissolved in a suitable solvent and treated with a stoichiometric amount of a sodium base at 0 to 50°C followed by trituration of the resultant solution in an antisolvent. An amorphous powder of pure montelukast sodium (I) is obtained.

As a suitable solvent for the saponification of the compound of the formula IX affording X one can use methanol, ethanol, n- or isopropanol, preferably methanol. As a suitable solvent for the isolation of compound of the formula X one can utilize the hydrocarbons such as hexane, n-heptane, cyclohexane, toluene, ethers such as diethyl ether, diisopropyl ether, methyl tertbutyl ether, THF, acetonitrile, preferably toluene.

As a suitable base for the saponification of the compound of the formula IX affording X one can use sodium hydroxide, potassium hydroxide, calcium hydroxide or magnesium hydroxide, preferably sodium hydroxide.

The saponification of the compound of the formula IX affording X is carried out at -10 to 80°C preferably 50°C.

As a suitable solvent for the dimetallation of the compound of the formula X affording XI one can utilize the hydrocarbons such as hexane, n-heptane, cyclohexane, toluene, ethers such as diethyl ether, diisopropyl ether, methyl tertbutyl ether, THF, acetonitrile, preferably THF.

As a suitable base for the dimetallation of the compound of the formula X affording X1 one can use n-butyl lithium, sodium hydride, sodium methoxide, potassium hydride, potassium methoxide, calcium hydride, magnesium oxide, phenyl sodium, preferably n-butyl lithium. The molar quantity of the base used for dimetallation can be varied between 0.95 and 0.99 moles but preferably 0.98 moles with respect to the compound of the formula IX.

The temperatures employed for the dimetallation of the compound of the formula X affording XI is carried out at -20 to 20°C preferably -10°C.

As a suitable solvent for the conversion of the compound of the formula II to the compound of the formula III, one can utilize the ethers such as dialkyl ethers, where alkyl connotes methyl, ethyl, n- & iso propyl, cyclic ethers such as THF, 1,4-dioxane, etc. More preferred ones are the cyclic ethers like tetrahydrofuran and 1,4-dioxane.

As a suitable reagent for the conversion of the compound of the formula II to the compound of the formula III, one can utilize the routinely available alkyl sulfonyl halide such as methanesulfonyl chloride, ethane sulfonyl chloride, propane sulfonyl chloride; methanesulfonyl bromide, ethane sulfonyl bromide, propane sulfonyl bromide; methanesulfonyl iodide, ethane sulfonyl iodide, propane sulfonyl iodide. More preferred ones are the methanesulfonyl chloride, ethane sulfonyl chloride.

The molar quantity of the alkyl sulfonyl halide used for the conversion of the compound of the formula II to the compound of the formula III can be varied between 1.0 and 1.5 but preferably 1.1-1.2 moles with respect to the compound of the formula II.

The temperatures employed for the conversion of the compound of the formula II to the compound of the formula III is carried out at -50 to 20°C preferably -20°C.

The reaction temperatures employed for the condensation between the compound of the formula III with the compound of the formula XI is carried out at -50 to 20°C preferably -10°C.

The molar quantity of the dimetallide XI used with respect to the compound of the formula III can be varied between 1.0-2.0 moles but preferably 1.4 to 1.5 moles with respect to the compound of the formula III.

As a suitable base for the conversion of the compound of the formula XI affording XII one can use either an achiral base like benzhydryl amine (aminodiphenylmethane) or the commercially available chirally pure bases such as α-methyl benzylamine, brucine, strychnine, quinine, cinchonidine, ephedrine, amphetamine, 3-nitro-α-methyl benzylamine, 4-nitro-α-methyl benzylamine, phenyl alinol, 1R,2R-2-amino-1,2-diphenylethanol, α-methyl naphthylethylamine, phenyl propanolamine etc. More preferred ones are the chirally pure bases. The molar quantity of the base used for this salt formation can be varied between 1.0 to 1.5 moles but preferably 1.1-1.2 moles with respect to the compound of the formula II.

As an organic solvent for purification of the compound of the formula XII one can utilize halogenated organic solvents, ethers, alkyl acetates, aromatic hydrocarbons, etc. More preferred are the alkyl acetates preferably ethyl acetate.

The organic solvent for dissolving the purified compound of the formula XII is selected from hydrocarbons such as hexane, n-heptane, cyclohexane, toluene, ethers such as diethyl ether, diisopropyl ether, methyl tertbutyl ether, ketones such as acetone, methyl ethyl ketone or methyl isobutyl ketone or esters such as methyl acetate, ethyl acetate or n-butyl acetate. More preferred one is toluene.

As a base for generating the sodium salt one can utilize the alkali metal hydroxides such as sodium hydroxide, the alkali metal carbonates such as sodium carbonate, the alkali metal bicarbonates such as sodium bicarbonate, alkali metal acetates such as sodium acetate, or alkali metal alkoxides such as sodium methoxide. More preferred one is sodium methoxide.

The suitable antisolvent for precipitating the compound of the formula I is selected from hydrocarbons such as hexane, n-heptane, cyclohexane, toluene, ethers such as diethyl ether, diisopropyl ether, methyl tertbutyl ether, ketones such as acetone, methyl ethyl ketone or methyl isobutyl ketone or esters such as methyl acetate, ethyl acetate or n-butyl acetate, preferably n-heptane.

The process does not proceed via the dilithio salt. The process of the present invention does not utilize any isolated mesylate. The process of the present invention does not employ the dicyclohexylamine salt as an intermediate. The process of the present invention does not utilize any freeze dryer for the isolation of the compound of the formula I. The process of the present invention does not proceed via the montelukast free acid.

The process of the invention does not utilize solvents such as acetonitrile during the final stages of crystallization, which have a stringent limit in ICH.

All the above collectively make the process economically more viable providing high yields and high purities for the final product.

### EXPERIMENTAL SECTION

### HPLC method for reaction monitoring:

Column: Cosmosil silica, 250 x 4.6 mm, 5.0 µ; wavelength: 280 n.m; injection volume: 10µL; Column temperature: 30°C; Run time: 30 min; Mobile phase: Hexane, dioxane & THF in the ratio of 85:15:2 and degassed.

### EXAMPLE 1

### Sodium 1-(mercaptomethyl)-cyclopropane acetate:

A solution of methyl 1-(mercaptomethyl)-cyclopropane acetate (50 gm, 0.31 mol) (IX) in methanol (250 ml) was treated with sodium hydroxide solution (62.0 gm in 200 ml distilled water) and stirred at 45°C for 2 hrs. The hydrolysis was monitored by TLC and the reaction mass was concentrated to a residual mass, which was dissolved in 300 ml of water and pH adjusted to 4.0 and reaction was extracted with 200ml of toluene. Toluene extract was stripped of toluene. The residue containing (X) was slurried in cyclohexane and filtered under nitrogen atmosphere, washed with cyclohexane (50 ml x 2) and dried under vacuum at 35°C to afford 44.61 gm of sodium 1-(mercaptomethyl)-cyclopropane acetate.
Yield = 85% (of theory)
NMR (CDCl3): δ 2.13-2.32 (m,4H), 0.27-0.44 (m, 4H)
XRD: As per fig-1

### Montelukast α-methyl benzyl amine salt

A suspension of 17 gm sodium 1-(mercaptomethyl)-cyclopropane acetate (X) (0.101 mol) in 75 ml THF was cooled to -40°C. To this 64.4 ml of n-butyl lithium (0.099 mol) was added followed by maintaining the reaction mass at -20 to -40°C for an additional 30 min to provide a mass of XI.

Simultaneously in another assembly the alcohol (II) (33 gm, 0.072 mol) was dissolved in 330 ml methylene chloride. To this N-methyl morpholine (10.91 gm, 0.108 mol) was added and the reaction mass was thereafter cooled to -25°C. Mesyl chloride (III) (9.76 gm, 0.085 mol) dissolved in 20 ml methylene chloride was added to the reaction mass and the reaction was stirred at -15 to -20°C for 2 hrs. The reaction mass was monitored by HPLC to check that the unreacted II was below 1%. Thereafter the reaction mass was further cooled to -40°C, filtered and the filtrate was concentrated in vacuum and the residue was dissolved in 600 ml THF. The resultant clear solution was cooled to - 45°C. To this the simultaneously prepared XI mass was added and the reaction was maintained at -10 to -15°C for 12 hrs and the reaction was again monitored by HPLC to check that the unreacted III was below 2%. The reaction mass was thereafter poured in a mixture of 1000 ml each of ethyl acetate and water and acidified with acetic acid to pH of 3.5. The layers were separated and the organic layer was washed with 200 ml water followed by 100 ml of 20% sodium chloride. The ethyl acetate solution was treated with charcoal, filtered through celite and treated with (R)-(+)- α-methylbenzyl amine (9.6 gm, 0.079 mol) to afford the crude salt. The resultant salt was filtered and purified by crystallization from 200 ml ethyl acetate to afford 43.8 gm of the α-methylbenzyl amine salt of montelukast.
Yield: 86% (of theory)
M.P: 126-7°C.
IR: 3336, 1604, 1541, 1496
NMR: δ 8.12-8.14 (d, 1H), 8.04-8.05 (d, 1H), 7.81 (s, 1H), 7.61-7.64 (bs, 1H), 7.70-7.74 (dd, 2H), 7.60-7.64 (d, 1H), 7.10-7.52 (m, 13H), 4.12-4.13 (q, 1H), 4.01-4.04 (t, 1H), 3.11-3.13 (m, 1H), 2.92-2.95 (m, 1H), 2.64-2.68 (d, 1H), 2.20-2.65 (m, 5H), 1.60-1.62 (2s, 6H), 1.40-1.41 (d, 3H), 0.46-0.55 (m, 4H)
Assay (by HPLC): 98.4%
Water content (by Karl Fisher): 0.12%
XRD: As per Fig-2

### EXAMPLE-2

The procedure of example 1 was followed with 23.33 gm of cinchonidine instead of (R)-(+)- α-methylbenzyl amine and the isolated product dried at 40°C under vacuum to give 53.5gm of cinchonidine salt of montelukast.
Yield: 84.4% (of theory)
M.P: 98 to 105°C.
IR: 3238, 2924; 1606;1593;1377;838;759 cm⁻¹
NMR: δ 6.88-8.60 (m, 21H); 5.66-5.68 (m, 1H); 5.07-5.09 (d, 1H); 4.69-4.78 (t, 2H); 1.97-3.78 (m, 14H); 1.17-1.21 (m, 15 H); δ 0.15-0.55 (m, 4H)
Assay (by HPLC): 98.5%
Water content (by Karl Fisher): 0.15%
XRD: As per Fig-3

### EXAMPLE-3

The procedure of example 1 was followed with 25.71 gm of quinine instead of (R)-(+)-α-methylbenzyl amine and the isolated product dried at 40°C under vacuum to give 54.12 gm of quinine salt of montelukast.
Yield: 82.5% (of theory)
M.P: 80 to 90°C.
IR: 3069; 2924, 1606; 1593; 1433; 861;760 cm⁻¹
NMR: δ 6.88 to 8.45 (m, 20H); 5.57-5.91 (m, 1H); 5.06-5.09 (d, 1H); 4.69-4.79 (t, 3H); 1.99-3.03 (m, 11H); 1.17-1.18 (d, 12 H); 1.22 (d, 6H); 0.15-0.23 (m, 6H )
Assay (by HPLC): 98.1 %
Water content (by Karl Fisher): 0.18%
XRD: As per Fig-4

### EXAMPLE-4

The procedure of example 1 was followed with 26.5 gm of strychnine instead of (R)-(+)-α-methylbenzyl amine and the isolated product dried at 40°C under vacuum to give 55gm strychnine salt of montelukast.
Yield: 83% (of theory)
M.P: 76 to 85°C.
IR: 3415, 1672, 1595, 1480; 761.8 cm⁻¹
NMR: δ 6.81-8.19 (m, 19H); 5.5-5.6(d, 1H); 3.12-4.36(m, 5H); 1.93-2.87(m, 19H); 0.99-1.75 (m, 10H); 0.14-0.26 (m, 4H)
Assay (by HPLC): 98.6%
Water content (by Karl Fisher): 0.19%
XRD: As per Fig-5

### EXAMPLE-5

The procedure of example 1 was followed with 10.85 gm of (+) phenylpropanolamine instead of (R)-(+)- α-methylbenzyl amine and the isolated product dried at 40°C under vacuum to give 43.6 gm phenylpropanolamine salt of montelukast.
Yield: 82% (of theory)
M.P: 156 to 159°C.
Assay (by HPLC): 98.1%
Water content (by Karl Fisher): 0.67%
XRD: As per Fig-6

### EXAMPLE-6

The procedure of example 1 was followed with 14.51 gm of benzhydrylamine instead of (R)-(+)- α-methylbenzyl amine and the isolated product dried at 40°C under vacuum to give 47.33 gm benzhydrylamine salt of montelukast.
Yield: 85.4% (of theory)
M.P: 128 to 134°C.
IR: 3371, 2667, 1606, 1542, 1497,1451,837, 759 in cm⁻¹
NMR: δ 8.67 to δ 7.08 (25 H (m) Aromatic & olefinic) ; δ 5.08 (s,1H) ; δ 3.95 (s,2H); δ 3.15 to 2.13 (m, 11H); δ 1.41 (s, 6H); δ 0.81 to 0.32 (m, 4H)
Assay (by HPLC): 98.5%
Water content (by Karl Fisher): 0.77%
XRD: As per Fig-7

### Montelukast sodium salt

The α-methyl benzyl amine salt (30 gm, 0.042mol) was dissolved in 240 ml toluene and to the resultant solution 2.4 gm of sodium methoxide (0.044mol) was added and the contents stirred for 30 min at 25-30°C followed by addition of 1.5 gm charcoal. The mass was stirred at 25-30°C for 1 hr and filtered through celite. The clear filtrate was added drop-wise into 900 ml n-heptane. The product mass was stirred for an additional 10 min at 25-30°C and filtered. The product was dried at 50°C under vacuum to get 24 gm of montelukast sodium.
Yield: 93% (of theory)
Water content (by Karl Fisher): 1.3%
Assay (by HPLC): 99.6%
SOR: +98.12
XRD: As per Fig-8

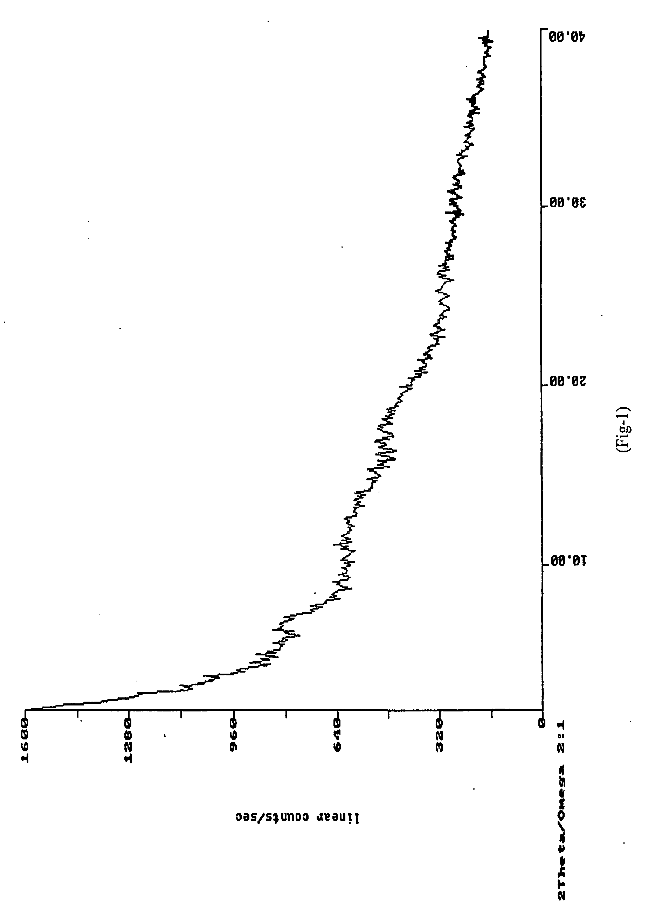

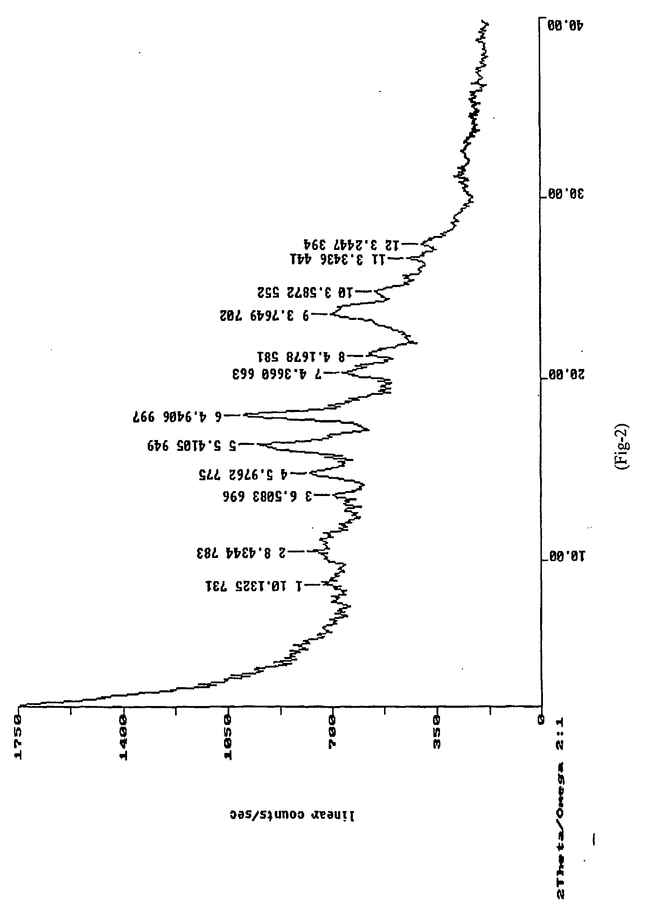

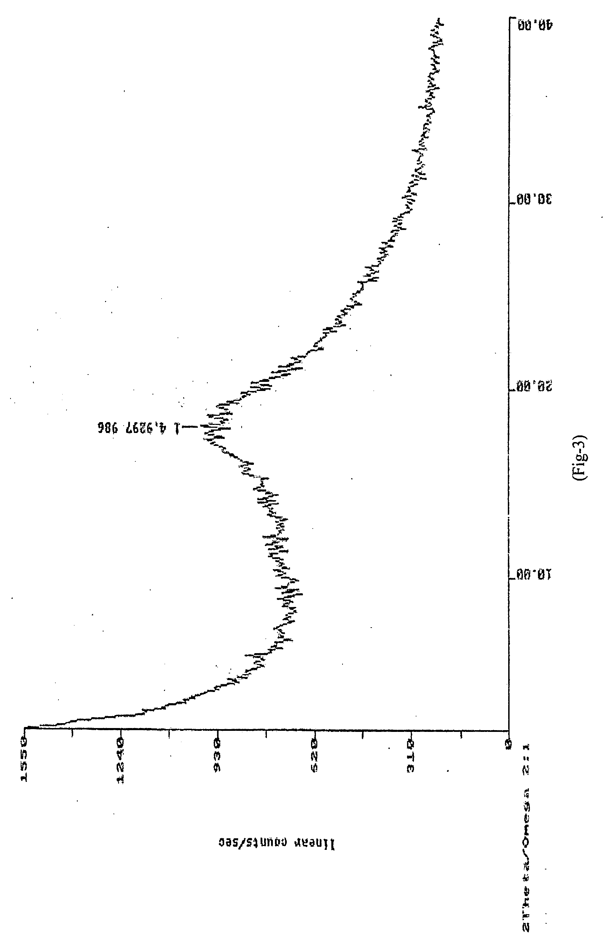

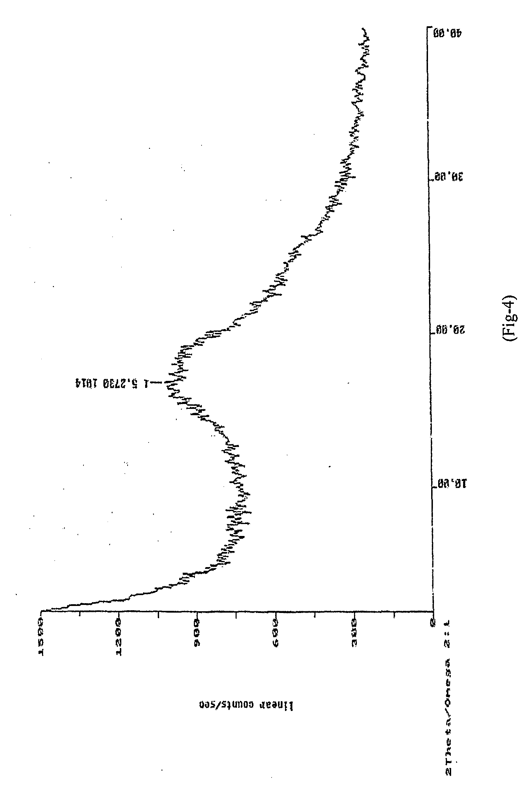

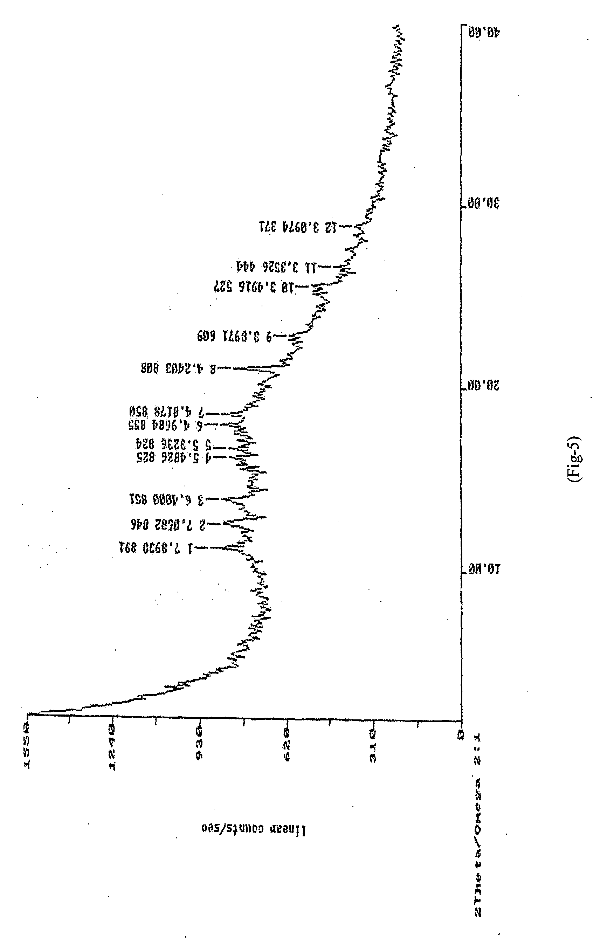

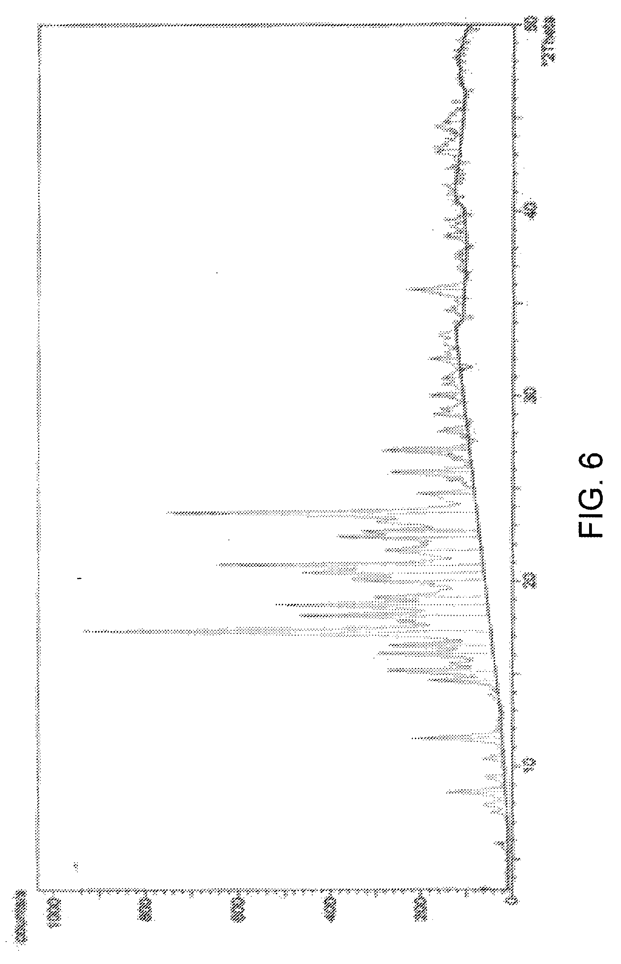

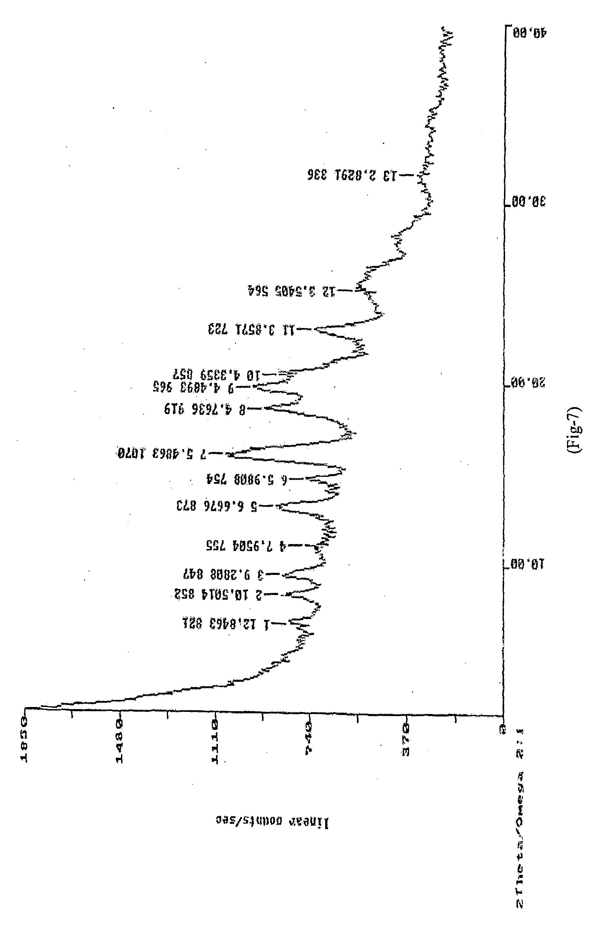

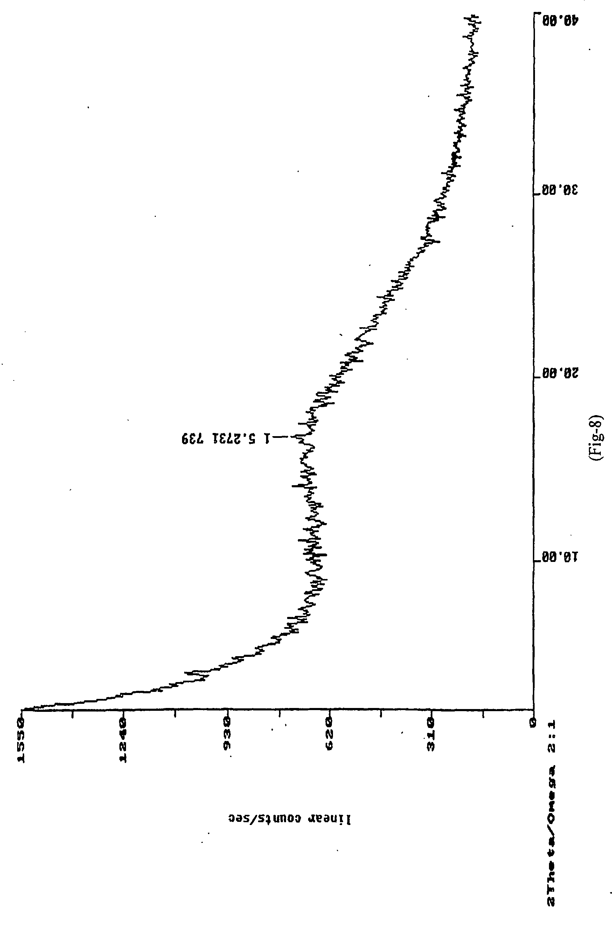

## Claims

1. A process for preparing 1-[[[(1R)-1-[3-[(1E)-2-(7-chloro-2-quinolinyl)ethenyl]phenyl]-3-[2-(1-hydroxy-1-methylethyl) phenyl] propyl] thio] methyl] cyclopropane acetic acid, sodium salt [Montelukast sodium (I)], the said process comprising steps of- 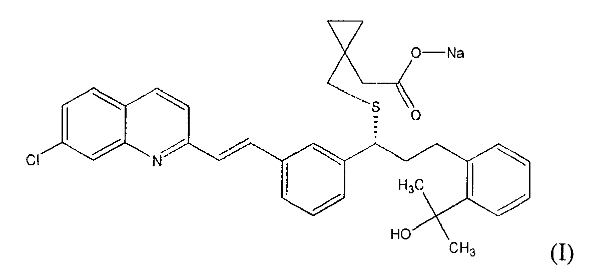
(a) treating methyl1-(mercaptomethyl)-cyclopropane acetate of formula (IX), in 2-10 volumes of a suitable solvent for saponification, along with alkali metal or alkaline earth metal hydroxide at a temperature ranging from 20 to 80°C affording the monometallide salt of the structure (X), which is isolated by concentrating the saponified mass in a suitable solvent followed by dehydrating the water formed during saponification by using a suitable base. 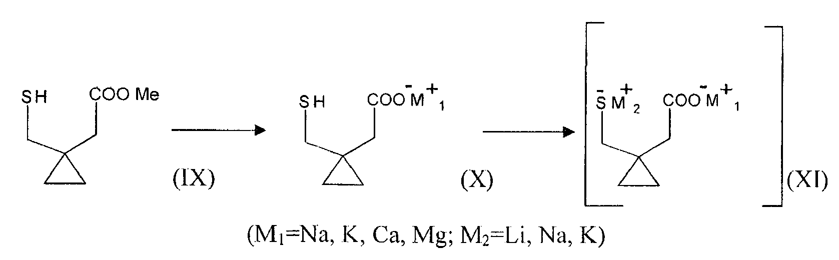
(b) treating the compound of the formula (X) with one molar equivalent of a metalide forming substance in a suitable solvent to get the dimetallide derivative of the formula (XI), at a temperature ranging from -20 to 20°C,
(c) treating the compound of the formula (II) with an alkylsulfonyl halide in a suitable solvent in presence of a base at a temperature ranging from -50 to 20°C followed by filtration and redissolution of the residue in a suitable solvent, 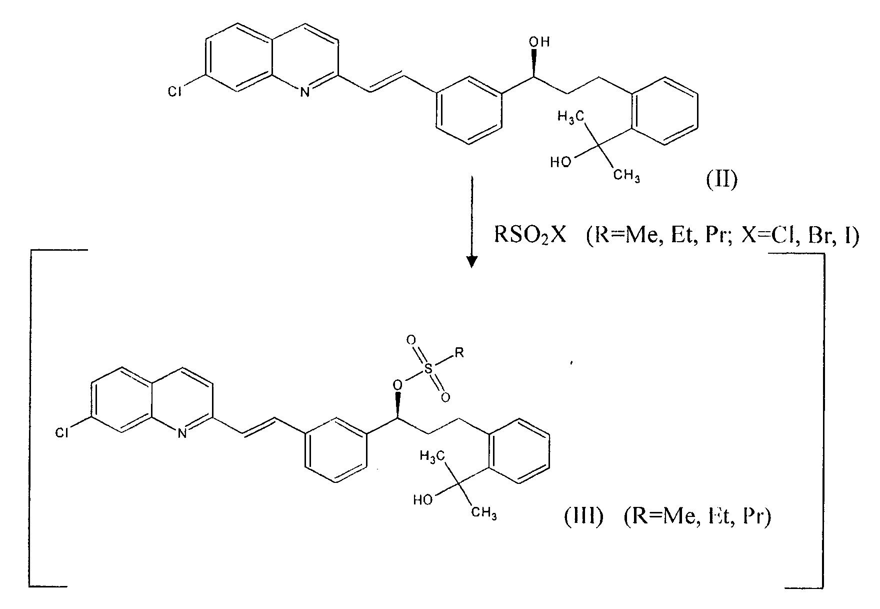
(d)mixing a solution of 2-(2-(3-(S)-(3-(2-(7-chloro-2-quinolinyl)-ethenyl)phenyl-3-alkylsulphonyloxypropyl)phenyl)-2-propanol (III) with a suspension of 1.1 to 1.5 moles of (XI) cooled to -20 to 0°C and maintained at that temperature for 8-20 hrs,
(e) extracting out the Montelukast, after adjusting the pH between 2-6, from the reaction mass by using a suitable solvent,
(f) reacting the Montelukast solution with a suitable base in a ratio of 1: (1.0-2.0) to afford the Montelukast salt (XII) 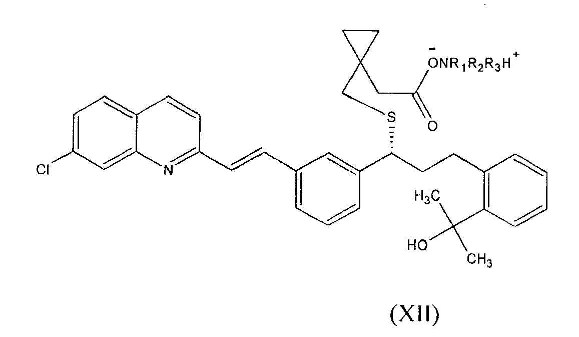
(g) purifying (XII) by crystallization from 3 to 5 volumes of a suitable solvent at 50-80°C and cooling to ambient temperature,
(h) dissolving purified (XII) in 8-10 volumes of a suitable solvent and treating in 1:1 I ratio with a suitable sodium base, at a temperature of 25 to 50°C,
(i) adding 25-50 volumes of an antisolvent and keeping the contents at 25 to 50°C,

2. A process as claimed in claim 1, wherein in step (a) the suitable solvent for saponification of the compound of the formula IX to a monometallide salt of formula (X) is selected from methanol, ethanol, n- or isopropanol.

3. The process as claimed in claim 1, wherein in step (a) the suitable solvent for the isolation of compound of the formula (X) is selected from the hydrocarbons such as hexane, n-heptane, cyclohexane, toluene, ethers such as diethyl ether, diisopropyl ether, methyl tertiary butyl ether, THF, acetonitrile.

4. The process as claimed in claim 1, wherein in step (a) the suitable base for the saponification of the compound of the formula (IX) affording (X) is selected from the group comprising sodium hydroxide, potassium hydroxide, calcium hydroxide or magnesium hydroxide.

5. The process as claimed in claim 1, wherein in step (a) the saponification of the compound of the formula (IX) affording (X) is carried out at 50°C.

6. The process as claimed in claim 1, wherein in step (b) the suitable solvent for the dimetallation of the compound of the formula (X) affording (XI) is selected from the hydrocarbons such as hexane, n-heptane, cyclohexane, toluene, ethers such as diethyl ether, diisopropyl ether, methyl tertiary butyl ether, THF, and acetonitrile.

7. The process as claimed in claim 1, wherein in step (b) the suitable reagent for the dimetallation of the compound of the formula (X) affording (XI) is selected from lithium forming substances such as n-, sec- & tert-butyl lithium, lithium hydride, sodium forming substance such as sodium hydride, sodium methoxide, phenyl sodium, potassium forming substance such as potassium hydride, potassium methoxide, calcium forming substance such as calcium hydride, magnesium forming substance such as magnesium oxide.

8. A process as claimed in claim 1, wherein in step (b) the temperatures employed for the dimetallation of the compound of the formula X affording XI is carried out at - 10°C.

9. A process as claimed in claim 1, wherein in step (c) the suitable solvent for the conversion of the compound of the formula (II) to the compound of the formula (III) is selected from the ethers such as dialkyl ethers, where alkyl connotes methyl, ethyl, n- & iso propyl, cyclic ethers such as THF and 1,4-dioxane.

10. The process as claimed in claim 1, wherein in step (c) the suitable alkyl sulfonyl halide for the conversion of the compound of the formula (II) to the compound of the formula (III) is selected from methanesulfonyl chloride, ethane sulfonyl chloride, propane sulfonyl chloride; methanesulfonyl bromide, ethane sulfonyl bromide, propane sulfonyl bromide; methanesulfonyl iodide, ethane sulfonyl iodide and propane sulfonyl iodide.

11. The process as claimed in claim 1, wherein in step (c) the molar quantity of the alkyl sulfonyl halide used for the conversion of the compound of the formula (II) to the compound of the formula (III) can be varied between 1.0 and 1.5.

12. The process as claimed in claim 1, wherein in step (c) the temperatures employed for the conversion of the compound of the formula (II) to the compound of the formula (III) is carried out at -20°C.

13. The process as claimed in claim 1, wherein in step (d) the reaction temperatures employed for the condensation between the compound of the formula (III) with the compound of the formula (XI) is carried out at -10°C.

14. The process as claimed in claim 1, wherein in step (d) the molar quantity of (XI) employed is 1.4-1.5 moles with respect to the compound of the formula (III).

15. The process as claimed in claim 1, wherein in step (e) the suitable solvent for extracting out the Montelukast, after adjusting the pH, from the reaction mass is selected from hydrocarbons such as hexane, n-heptane, cyclohexane, toluene, ethers such as diethyl ether, diisopropyl ether, methyl tertbutyl ether, or esters such as methyl acetate, ethyl acetate or n-butyl acetate.

16. The process as claimed in claim 1, wherein in step (e) the suitable pH is selected from 2 to 6 units.

17. The process as claimed in claim 1, wherein in step (f) the suitable base for the conversion of the compound of the formula (XI) affording (XII) is selected from either an achiral base like benzhydryl amine (aminodiphenylmethane) or the commercially available chirally pure bases such as α-methyl benzylamine, brucine, strychnine, quinine, cinchonidine, ephedrine, amphetamine, 3-nitro-α-methyl benzylamine, 4-nitro-α-methyl benzylamine, phenyl alinol, 1R,2R-2-amino-1,2-diphenylethanol, α-methyl naphthylethylamine and phenyl propanolamine.

18. The process as claimed in claim 1, wherein in step (f) the molar quantity of the base used for this salt formation can be varied between 1.1 to 1.2 moles with respect to the compound of the formula (II).

19. The process as claimed in claim 1, wherein in step (g) the organic solvent for purification of the compound of the formula (XII) is selected from halogenated organic solvents, ethers, alkyl acetates such as ethyl acetate and aromatic hydrocarbons.

20. The process as claimed in claim 1, wherein in step (h) the suitable solvent for dissolving the purified compound of the formula (XII) is selected from hydrocarbons such as hexane, n-heptane, cyclohexane, toluene, ethers such as diethyl ether, diisopropyl ether, methyl tertbutyl ether, ketones such as acetone, methyl ethyl ketone or methyl isobutyl ketone or esters such as methyl acetate, ethyl acetate or n-butyl acetate.

21. The process as claimed in claim 1, wherein in step (h) the suitable base for converting the purified compound of the formula (XII) to the sodium salt is selected from sodium hydroxide, sodium carbonate, sodium bicarbonate, sodium alkoxide, where alkoxide connotes methoxide, ethoxide, n- & iso- propoxide.

22. The process as claimed in claim 1, wherein in step (i) the suitable antisolvent for precipitating the compound of the formula 1 is selected from hydrocarbons such as hexane, n-heptane, cyclohexane, toluene, ethers such as diethyl ether, diisopropyl ether, methyl tertbutyl ether, ketones such as acetone, methyl ethyl ketone or methyl isobutyl ketone or esters such as methyl acetate, ethyl acetate or n-butyl acetate.

## Patentansprüche

1. Verfahren zur Herstellung des Natriumsalzes von 1-[[[(1*R*)-1-[3-[(1*E*)-2-(7-Chlor-2-chinolinyl)ethenyl]phenyl]-3-[2-(1-hydroxy-1-methylethyl)phenyl]propyl]thio]methyl]-cyclopropanessigsäure [Natrium-Montelukast (I)] 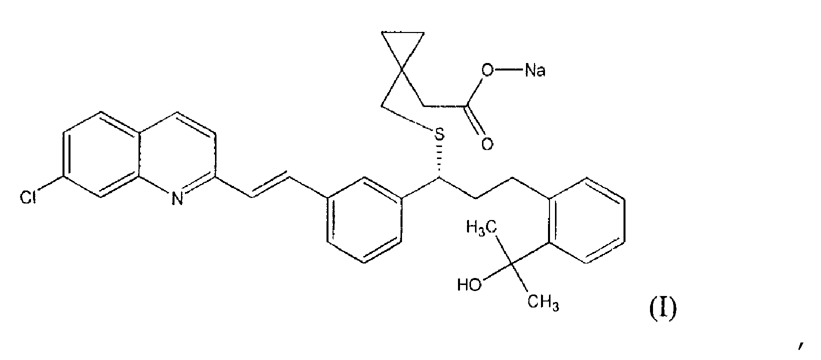 wobei das Verfahren die Schritte umfasst:
(a) Behandeln von Methyl 1-(mercaptomethyl)-cyclopropanacetat der Formel (IX) in 2-10 Volumina eines geeigneten Lösungsmittels zur Verseifung, zusammen mit Alkalimetall- oder Erdalkalimetallhydroxid bei einer von 20 bis 80°C reichenden Temperatur, was das Monometallidsalz der Struktur (X) liefert, welches durch Konzentrieren der verseiften Masse in einem geeigneten Lösungsmittel, gefolgt vom Dehydrieren des während der Verseifung unter Verwendung einer geeigneten Base gebildeten Wassers, isoliert wird, 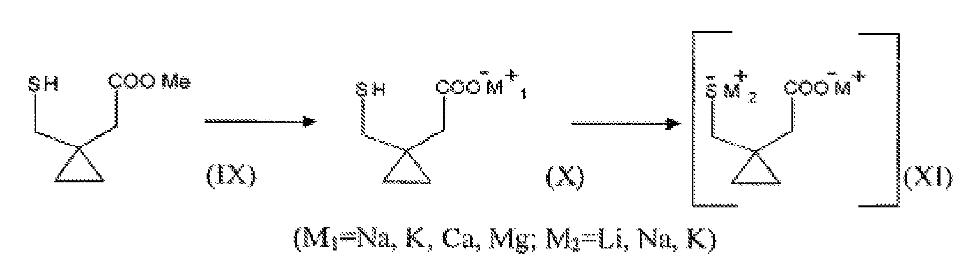
(b) Behandeln der Verbindung der Formel (X) mit einem Moläquivalent einer Metallid-bildenden Substanz in einem geeigneten Lösungsmittel bei einer von -20 bis 20°C reichenden Temperatur, um das Dimetallidderivat der Formel (XI) zu erhalten,
(c) Behandeln der Verbindung der Formel (II) mit einem Alkylsulfonylhalogenid in einem geeigneten Lösungsmittel in der Gegenwart einer Base bei einer von -50 bis 20°C reichenden Temperatur, gefolgt von einer Filtration und Wiederauflösen des Rückstands in einem geeigneten Lösungsmittel, 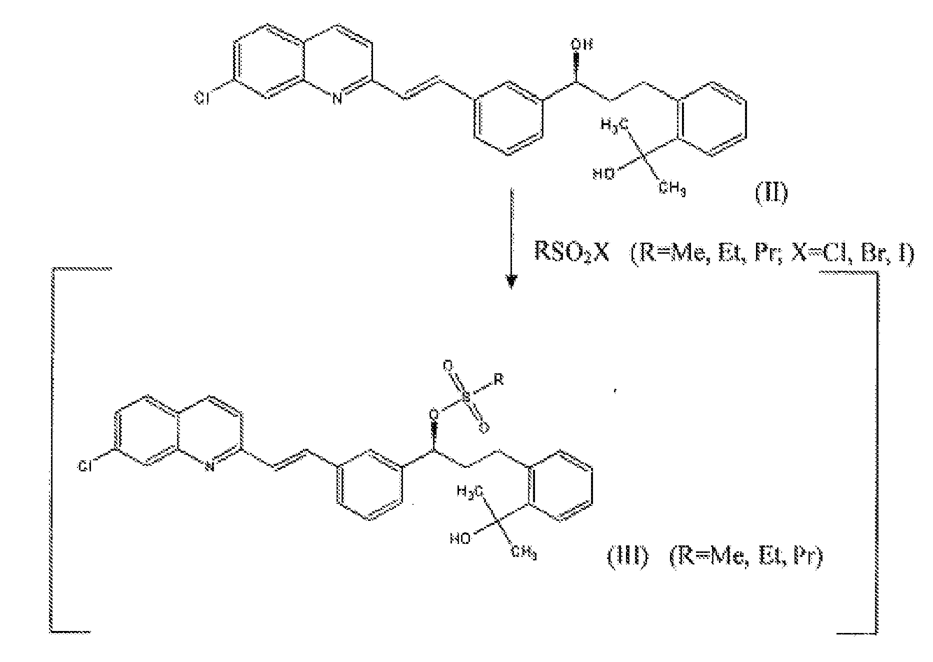
(d) Mischen einer Lösung von 2-(2-(3-(*S*)-(3-(2-(7-Chlor-2-chinolinyl)-ethenyl)phenyl-3-alkylsulphonyloxypropyl)phenyl)-2-propanol (III) mit einer Suspension von 1,1 bis 1,5 Mol (XI), was auf -20 bis 0°C gekühlt wird und für 8-20 Stunden auf dieser Temperatur gehalten wird,
(e) Herausextrahieren des Montelukast aus der Reaktionsmasse, nach Einstellen des pHs zwischen 2-6, unter Verwendung eines geeigneten Lösungsmittels,
(f) Umsetzen der Montelukastlösung mit einer geeigneten Base in einem Verhältnis von 1:(1,0-2,0), um das Montelukastsalz (XII) zu liefern, 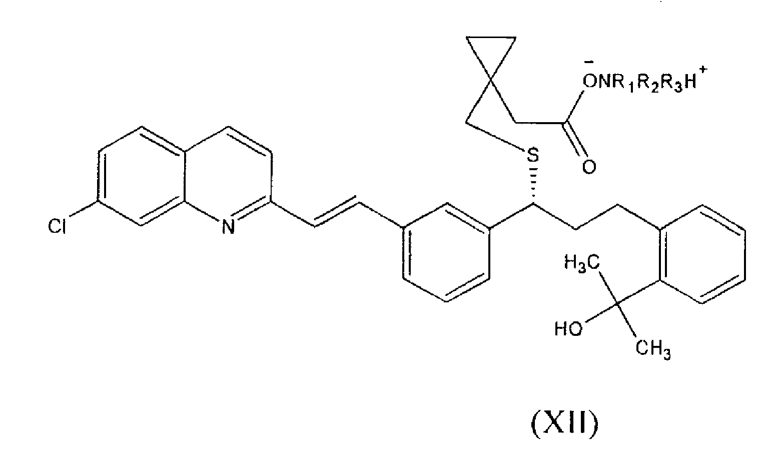
(g) Reinigen von (XII) durch Kristallisation aus 3 bis 5 Volumina eines geeigneten Lösungsmittels bei 50-80°C und Kühlen auf Umgebungstemperatur,
(h) Lösen des gereinigten (XII) in 8-10 Volumina eines geeigneten Lösungsmittels und Behandeln mit einer geeigneten Natriumbase im Verhältnis 1:1 bei einer Temperatur von 25 bis 50°C,
(i) Zufügen von 25-50 Volumina eines Antisolvens und Halten des Inhalts bei 25 bis 50°C.

2. Verfahren wie in Anspruch 1 beansprucht, wobei in Schritt (a) das geeignete Lösungsmittel zur Verseifung der Verbindung der Formel (IX) zu einem Monometallidsalz der Formel (X) aus Methanol, Ethanol, n- oder Isopropanol ausgewählt wird.

3. Verfahren wie in Anspruch 1 beansprucht, wobei in Schritt (a) das geeignete Lösungsmittel zum Isolieren der Verbindung der Formel (X) aus Kohlenwasserstoffen wie Hexan, n-Heptan, Cyclohexan, Toluol, Ethern wie Diethylether, Diisopropylether, tert-Butylmethylether, THF, Acetonitril ausgewählt wird.

4. Verfahren wie in Anspruch 1 beansprucht, wobei in Schritt (a) die geeignete Base zur (X) liefernden Verseifung der Verbindung der Formel (IX) aus der Natriumhydroxid, Kaliumhydroxid, Calciumhydroxid oder Magnesiumhydroxid umfassenden Gruppe ausgewählt wird.

5. Verfahren wie in Anspruch 1 beansprucht, wobei in Schritt (a) die (X) liefernde Verseifung der Verbindung der Formel (IX) bei 50°C durchgeführt wird.

6. Verfahren wie in Anspruch 1 beansprucht, wobei in Schritt (b) das geeignete Lösungsmittel zur (XI) liefernden Dimetallierung der Verbindung der Formel (X) aus Kohlenwasserstoffen wie Hexan, n-Heptan, Cyclohexan, Toluol, Ethern wie Diethylether, Diisopropylether, tert-Butylmethylether, THF und Acetonitril ausgewählt wird.

7. Verfahren wie in Anspruch 1 beansprucht, wobei in Schritt (b) das geeignete Reagens zur (XI) liefernden Dimetallierung der Verbindung der Formel (X) aus Lithiumbildenden Substanzen wie n-, sec- und tert-Butyllithium, Lithiumhydrid, Natrium-bildenden Substanzen wie Natriumhydrid, Natriummethoxid, Phenylnatrium, Kaliumbildenden Substanzen wie Kaliumhydrid, Kaliummethoxid, Calcium-bildenden Substanzen wie Calciumhydrid und Magnesiumbildenden Substanzen wie Magnesiumoxid ausgewählt wird.

8. Verfahren wie in Anspruch 1 beansprucht, wobei in Schritt (b) die für die (XI) liefernde Dimetallierung der Verbindung der Formel X angewendeten Temperaturen bei -10°C liegen.

9. Verfahren wie in Anspruch 1 beansprucht, wobei in Schritt (c) das geeignete Lösungsmittel für die Umwandlung der Verbindung der Formel (II) in die Verbindung der Formel (III) aus Ethern wie Dialkylethern, wobei Alkyl Methyl, Ethyl, n- und Isopropyl bedeutet, zyklischen Ethern wie THF und 1,4-Dioxan ausgewählt wird.

10. Verfahren wie in Anspruch 1 beansprucht, wobei in Schritt (c) das geeignete Alkylsulfonylhalogenid für die Umwandlung der Verbindung der Formel (II) in die Verbindung der Formel (III) aus Methansulfonylchlorid, Ethansulfonylchlorid, Propansulfonylchlorid, Methansulfonylbromid, Ethansulfonylbromid, Propansulfonylbromid, Methansulfonyliodid, Ethansulfonyliodid und Propansulfonyliodid ausgewählt wird.

11. Verfahren wie in Anspruch 1 beansprucht, wobei in Schritt (c) die Molmenge des für die Umwandlung der Verbindung der Formel (II) in die Verbindung der Formel (III) verwendeten Alkylsulfonylhalogenids zwischen 1,0 und 1,5 variiert werden kann.

12. Verfahren wie in Anspruch 1 beansprucht, wobei in Schritt (c) die für die Umwandlung der Verbindung der Formel (II) in die Verbindung der Formel (III) angewendeten Temperaturen bei -20°C liegen.

13. Verfahren wie in Anspruch 1 beansprucht, wobei in Schritt (d) die für die Kondensierung zwischen der Verbindung der Formel (III) und der Verbindung der Formel (XI) angewendet Reaktionstemperaturen bei -10°C liegen.

14. Verfahren wie in Anspruch 1 beansprucht, wobei in Schritt (d) die angewendete Molmenge von (XI) 1,4-1,5 Mol in Bezug auf die Verbindung der Formel (III) beträgt.

15. Verfahren wie in Anspruch 1 beansprucht, wobei in Schritt (e) das geeignete Lösungsmittel zum Herausextrahieren des Montelukast aus der Reaktionsmasse nach Einstellen des pHs aus Kohlenwasserstoffen wie Hexan, n-Heptan, Cyclohexan, Toluol, Ethern wie Diethylether, Diisopropylether, tert-Butylmethylether oder Estern wie Methylacetat, Ethylacetat oder n-Butylacetat ausgewählt wird.

16. Verfahren wie in Anspruch 1 beansprucht, wobei in Schritt (e) der geeignete pH 2 bis 6 beträgt.

17. Verfahren wie in Anspruch 1 beansprucht, wobei in Schritt (f) die geeignete Base zur Umwandlung der Verbindung der Formel (XI), die (XII) liefert, entweder aus einer achiralen Base wie Benzhydrylamin (Aminodiphenylmethan) oder einer kommerziell erhältlichen, chiral reinen Base wie α-Methylbenzylamin, Brucin, Strychnin, Quinin, Cinchonidin, Ephedrin, Amphetamin, 3-Nitro-α-methylbenzylamin, 4-Nitro-α-methylbenzylamin, Phenylalinol, 1R,2R-2-amino-1,2-diphenyl-ethanol, α-Methylnaphthylethylamin und Phenylpropanolamin ausgewählt wird.

18. Verfahren wie in Anspruch 1 beansprucht, wobei in Schritt (f) die Molmenge der für diese Salzbildung verwendeten Base zwischen 1,1 bis 1,2 Mol in Bezug auf die Verbindung der Formel (II) variiert werden kann.

19. Verfahren wie in Anspruch 1 beansprucht, wobei in Schritt (g) das organische Lösungsmittel zur Reinigung der Verbindung der Formel (XII) aus halogenierten organischen Lösungsmitteln, Ethern, Alkylacetaten wie Ethylacetat und aromatischen Kohlenwasserstoffen ausgewählt wird.

20. Verfahren wie in Anspruch 1 beansprucht, wobei in Schritt (h) das geeignete Lösungsmittel zum Lösen der gereinigten Verbindung der Formel (XII) aus Kohlenwasserstoffen wie Hexan, n-Heptan, Cyclohexan, Toluol, Ethern wie Diethylether, Diisopropylether, tert-Butylmethylether, Ketonen wie Aceton, Methylethylketon oder Methylisobutylketon oder Estern wie Methylacetat, Ethylacetat oder n-Butylacetat ausgewählt wird.

21. Verfahren wie in Anspruch 1 beansprucht, wobei in Schritt (h) die geeignete Base zum Umwandeln der gereinigten Verbindung der Formel (XII) in das Natriumsalz aus Natriumhydroxid, Natriumcarbonat, Natriumbicarbonat, Natriumalkoxid, wobei Alkoxid Methoxid, Ethoxid, n- und iso-Propoxid bezeichnet, ausgewählt wird.

22. Verfahren wie in Anspruch 1 beansprucht, wobei in Schritt (i) das geeignete Antisolvens zum Fällen der Verbindung der Formel (I) aus Kohlenwasserstoffen wie Hexan, n-Heptan, Cyclohexan, Toluol, Ethern wie Diethylether, Diisopropylether, tert-Butylmethylether, Ketonen wie Aceton, Methylethylketon oder Methylisobutylketon oder Estern wie Methylacetat, Ethylacetat oder n-Butylacetat ausgewählt wird.

## Revendications

1. Procédé de préparation du sel de sodium de l'acide 1-[[[([3-[(1E)-2-(7-chloro-2-quinolinyl)-éthényl]phényl]-3-[2-(1-hydroxy-1-méthyléthyl)-phényl]-propyl]thio]méthyl]cyclopropane-acétique [montélukast sodique (I)], ledit procédé comprenant les étapes suivantes: 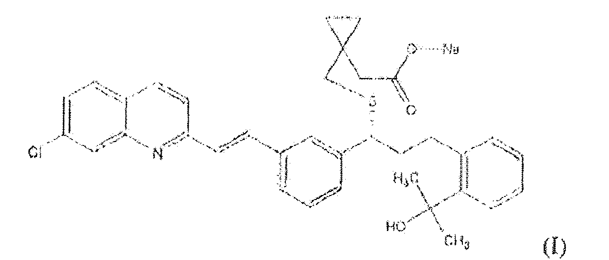
(a) le traitement du 1-(mercaptométhyl)-cyclopropane acétate de méthyle de formule (IX), dans 2 à 10 volumes de solvant approprié pour une saponification, avec un hydroxyde de métal alcalin ou de métal alcalino-terreux à une température allant de 20°C à 80°C pour donner le sel monométallique de structure (X) ; lequel est isolé par concentration de la masse saponifiée dans un solvant approprié puis par déshydratation de l'eau formée lors de la saponification au moyen d'une base appropriée, 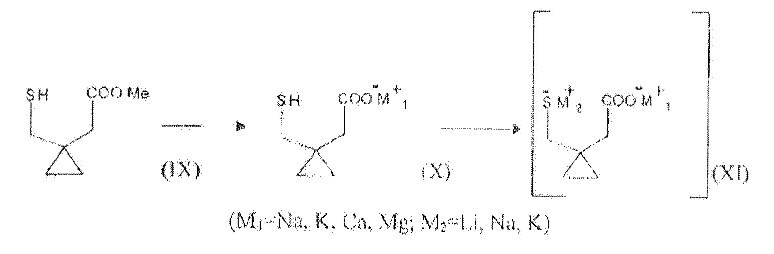
(b) le traitement du composé de formule (X) avec un équivalent molaire d'une substance formant un sel métallique dans un solvant approprié pour obtenir le dérivé dimétallique de formule (XI), à une température allant de -20°C à 20°C,
(c) le traitement du composé de formule (II) avec un halogénure d'alkylsulfonyle dans un solvant approprié en présence d'une base à une température allant de -50°C à 20°C, puis la filtration et la redissolution du résidu dans un solvant appropriée, 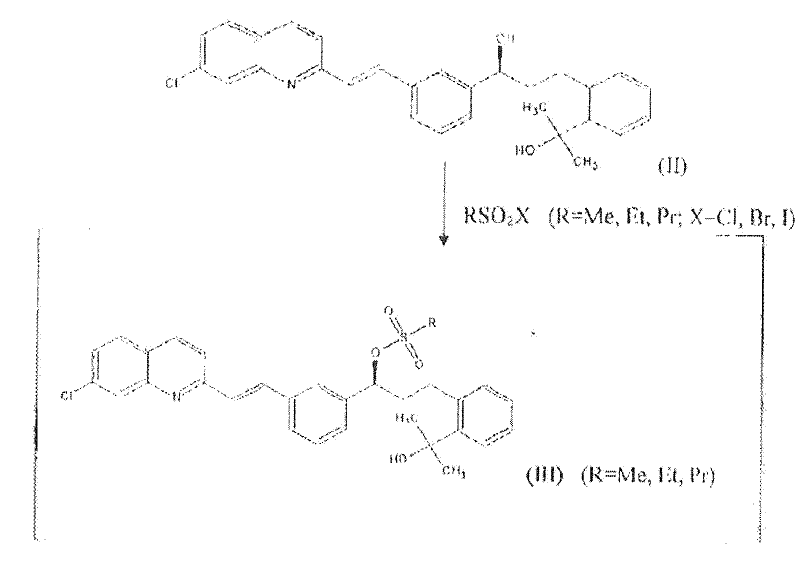
(d) le mélange d'une solution de 2-(2-(3-(S)-(3-(2-(7-chloro-2-quinolinyl) éthényl)phényl-3-alkylsulfonyloxypropyl)phényl)-2-propanol (III) avec une suspension contenant de 1,1 à 1,5 mole de (XI) refroidie à -20°C à 0°C et maintenue à cette température pendant 8 heures à 20 heures,
(e) l'extraction du montélukast, après ajustement du pH entre 2 et 6, à partir de la masse réactionnelle au moyen d'un solvant approprié,
(f) la réaction de la solution de montélukast avec une base appropriée dans un rapport de 1 : (1,0 à 2,0) pour donner le sel de montélukast (XII), 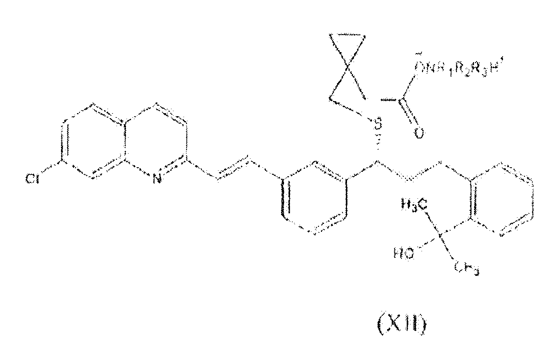
(g) la purification de (XII) par cristallisation dans 3 à 5 volumes d'un solvant approprié à une température allant de 50°C à 80°C et refroidissement jusqu'à température ambiante,
(h) la dissolution de (XII) purifié dans 8 à 10 volumes d'un solvant approprié et le traitement dans un rapport 1 : 1 avec une base sodique appropriée, à une température allant de 25°C à 50°C,
(i) l'ajout de 25 à 50 volumes d'un antisolvant et le maintien du contenu à une température de 25°C à 50°C.

2. Le procédé selon la revendication 1, dans lequel dans l'étape (a), le solvant approprié pour la saponification du composé de formule IX en sel monométallique de formule (X) est choisi parmi le méthanol, l'éthanol, le n-propanol et l'isopropanol.

3. Le procédé selon la revendication 1, dans lequel dans l'étape (a), le solvant approprié pour l'isolement du composé de formule (X) est choisi parmi des hydrocarbures tels que l'hexane, le n-heptane, le cyclohexane, le toluène, des éthers tels que le diéthyl éther, le diisopropyl éther, le méthyl tert-butyl éther, le THF, l'acétonitrile.

4. Le procédé selon la revendication 1, dans lequel dans l'étape (a), la base appropriée pour la saponification du composé de formule (IX) pour obtenir (X) est choisie dans le groupe comprenant l'hydroxyde de sodium, l'hydroxyde de potassium, l'hydroxyde de calcium et l'hydroxyde magnésium.

5. Le procédé selon la revendication 1, dans lequel dans l'étape (a), la saponification du composé de formule (IX) pour donner (X) est réalisée à 50°C.

6. Le procédé selon la revendication 1, dans lequel dans l'étape (b), le solvant approprié pour la dimétallation du composé de formule (X) pour donner (XI) est choisi parmi des hydrocarbures tels que l'hexane, le n-heptane, le cyclohexane, le toluène, des éthers tels que le diéthyl éther, le diisopropyl éther, le méthyl tert-butyl éther, le THF et l'acétonitrile.

7. Le procédé selon la revendication 1, dans lequel dans l'étape (b), le réactif approprié pour la dimétallation du composé de formule (X) pour donner (XI) est choisi parmi des substances formant du lithium telles que le n-, sec- et tert-butyl lithium, l'hydrure de lithium, une substance formant du sodium telle que l'hydrure de sodium, le méthanolate de sodium, le phényle sodique, une substance formant du potassium telle que l'hydrure de potassium, le méthanolate de potassium, une substance formant du calcium telle que l'hydrure de calcium, une substance formant du magnésium telle que l'oxyde de magnésium.

8. Le procédé selon la revendication 1, dans lequel dans l'étape (b), la température utilisée pour la dimétallation du composé de formule X pour donner XI est -10°C.

9. Le procédé selon la revendication 1, dans lequel dans l'étape (c), le solvant approprié pour la conversion du composé de formule (II) en composé de formule (III) est choisi parmi des éthers tels que les dialkyléthers, allyle indiquant le méthyle, l'éthyle, le n-propyle ou l'isopropyle, des éthers cycliques tels que le THF et le 1,4-dioxanne.

10. Le procédé selon la revendication 1, dans lequel dans l'étape (c), l'halogénure d'alkylsulfonyle approprié pour la conversion du composé de formule (II) en composé de formule (III) est choisi parmi le chlorure de méthanesulfonyle, le chlorure d'éthane sulfonyle, le chlorure de propanesulfonyle ; le bromure de méthanesulfonyle, le bromure d'éthane sulfonyle, le bromure de propanesulfonyle ; l'iodure de méthanesulfonyle, l'iodure d'éthanesulfonyle et l'iodure de propane sulfonyle.

11. Le procédé selon la revendication 1, dans lequel dans l'étape (c), la quantité molaire de l'halogénure d'alkylsulfonyle utilisée pour la conversion du composé de formule (II) en composé de formule (III) peut varier entre 1,0 et 1,5.

12. Le procédé selon la revendication 1, dans lequel dans l'étape (c), la température utilisée pour la conversion du composé de formule (II) en composé de formule (III) est -20°C.

13. Le procédé selon la revendication 1, dans lequel dans l'étape (d), la température réactionnelle utilisée pour la condensation entre le composé de formule (III) et le composé de formule (XI) est -10°C.

14. Le procédé selon la revendication 1, dans lequel dans l'étape (d), la quantité molaire de (XI) utilisée est de 1,4 à 1,5 mole par rapport au composé de formule (III).

15. Le procécé selon la revendication 1, dans lequel dans l'étape (e), le solvant approprié pour l'extraction du montélukast, après ajustement du pH, à partir de la masse réactionnelle est choisi parmi des hydrocarbures tels que l'hexane, le n-heptane, le cyclohexane, le toluène, des éthers tels que le diéthyl éther, le diisopropyl éther, le méthyl tert-butyl éther, ou des esters tels que l'acétate de méthyle, l'acétate d'éthyle ou l'acétate de n-butyle.

16. Le procédé selon la revendication 1, dans lequel dans l'étape (e), le pH approprié va de 2 à 6 unités.

17. Le procédé selon la revendication 1, dans Lequel dans l'étape (f), la base appropriée pour la conversion du composé de formule (XI) pour donner (XII) est choisie parmi une base achirale comme la benzhydryl amine (aminodiphénylméthane) ou des bases chiralement pures disponibles dans le commerce, telles que l'α-méthyl benzylamine, la brucine, la strychnine, la quinine, la cinchonidine, l'éphédrine, l'amphétamine, la 3-nitro-α-méthyl benzylamine, la 4-nitro-a-méthyl benzylamine, le phényl alinol, le 1R, 2R-2-amino-1,2-diphényléthanol, l'α-méthyl naphtyléthylamine et la phényl propanolamine.

18. Le procédé selon la revendication 1, dans lequel dans l'étape (f), la quantité molaire de la base utilisée pour la formation de ce sel peut varier entre 1,1 et 1,2 mole par rapport au composé de formule (II).

19. Le procédé selon la revendication 1, dans lequel dans l'étape (g), le solvant organique pour la purification du composé de formule (XII) est choisi parmi les solvants organiques halogénés, les acétates d'alkyle tels que l'acétate d'éthyle et les hydrocarbures aromatiques.

20. Le procédé selon la revendication 1, dans s lequel dans l'étape (h), le solvant approprié pour la dissolution du composé purifié de formule (XII) est choisi parmi des hydrocarbures tels que l'hexane, le n-heptane, le cyclohexane, le toluène, des éthers tels que le diéthyl éther, le diisopropyl éther, le méthyl tert-butyl éther, des cétones telles que l'acétone, la méthyl éthyl cétone ou la méthyl isobutyl cétone, ou des esters tels que l'acétate de méthyle, l'acétate d'éthyle ou l'acétate de n-butyle.

21. Le procédé selon la revendication 1, dans lequel dans l'étape (h), la base appropriée pour la conversion du composé purifié de formule (XII) en sel de sodium est choisie parmi l'hydroxyde sodium, le carbonate de sodium, le bicarbonate de sodium, un alcoxyde de sodium, alcoolate indiquant le méthanolate, l'éthanolate, le n-iso propanolate ou l'isopropanolate.

22. Le procédé selon la revendication 1, dans lequel 1 dans s l'étape (i), l'antisolvant approprié pour la précipitation du composé de formule I est choisi parmi des hydrocarbures tels que l'hexane, le n-heptane, le cyclohexane, le toluène, des éthers tels que le diéthyl éther, le diisopropyl éther, le méthyl tert--butyl éther, des cétones telles que l'acétone, la méthyl ethyl cétone ou la méthyl isobutyl cétone, ou des esters tels que l'acétate de méthyle, l'acétate d'éthyle ou l'acétate de n-butyle.
